(19) Europäisches Patentamt
European Patent Office
Office européen des brevets

(11) Publication number: 0 233 393 B1

(12) **EUROPEAN PATENT SPECIFICATION**

(45) Date of publication of the patent specification: **27.06.90**

(51) Int. Cl.[5]: **C07C 233/02**, C07C 233/05, C07C 231/00, B01J 27/24

(21) Application number: **86301163.1**

(22) Date of filing: **19.02.86**

(54) **Method for producing monoethylformamide and dimethylformamide.**

(43) Date of publication of application: **26.08.87 Bulletin 87/35**

(45) Publication of the grant of the patent: **27.06.90 Bulletin 90/26**

(84) Designated Contracting States: **AT BE CH DE FR GB IT LI LU NL SE**

(56) References cited:
**DE-C- 1 000 020**

**CHEMICAL ABSTRACTS, vol. 79, no. 25, 24th December 1973, page 285, left-hand column, abstract no. 146019r, Columbus, Ohio, US; S. SENOO et al.: "Aliphatic tertiary amides", & JP - A - 73 28417 (ASAHI CHEMICAL INDUSTRY)**
**CHEMICAL ABSTRACTS, vol. 93, no. 3, 21st July 1980, page 614, left-hand column, abstract no. 25372f, Columbus, Ohio, US; H. KASHIWAGI et al.: "N-Alkylation of amines and amides catalyzed by ammonium halides", & NIPPON KAGAKU KAISHI 1980, (2), 279-281"lladium(II)-Komplexen mit Silbertetrafluoroborat zur Dimerisation von Methylacrylat" 000**
**CHEMICAL ABSTRACTS, vol. 57, no. 13, 24th December 1962, columns 16392i-16393b, Columbus, Ohio, US; Y. TAKEZAKI et al.: "One-step synthesis of dimethylformamide under high pressure" & KOGYO KAGAKU ZASSHI 1960, 63, 1739-1745 (Cat. A,D)**

(73) Proprietor: **E.I. DU PONT DE NEMOURS AND COMPANY, 1007 Market Street, Wilmington Delaware 19898(US)**

(72) Inventor: **Bellis, Harold Edward, 1 Hillvale Circle, Wilmington, Delaware 19808(US)**

(74) Representative: **Barnard, Eric Edward et al, BROOKES & MARTIN High Holborn House 52/54 High Holborn, London WC1V 6SE(GB)**

(56) References cited: (continuation)
**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 233 393 B1

ACTORUM AG

## Description

### Technical Field

This invention relates to a process for preparing monomethylformamide (MMF) and dimethylformamide (DMF). It is more particularly directed to such a process in which MMF and DMF are catalytically prepared from formamide and methanol.

### Background and Summary of the Invention

DMF is a commodity in the chemical industry, widely used as a solvent. One of the common methods of preparing it is by the reaction of dimethylamine and carbon monoxide. This requires either a large plant for preparing the dimethylamine starting material, with its attendant huge investment (small plants are ordinarily uneconomical), or the large-scale purchase of dimethylamine on the open market, neither of which is satisfactory from an economic standpoint.

A satisfactory alternative would be a method whereby MMF and DMF are prepared by the reaction of formamide and methanol. Methanol is plentiful and inexpensive, and formamide is easily prepared from ammonia and carbon monoxide, both readily available and cheap.

The reaction of formamide and methanol to form MMF and DMF is known (See, for example "A Study on High Pressure One-Step Synthesis of Dimethylformamide," Takezaki, Kitahama, Suzuki, Sugita and Yuasa, Kogyo Kagaku Zasshi 63, 1739-45 (1960), but as described in the prior art is not practical on a commercial scale because of low yields.

We have now found that MMF and DMF can be prepared, easily and in good yield, by the catalytic reaction of formamide and methanol, using a particular quaternary ammonium compound as the catalyst.

### Detailed Description of the Invention

The catalyst used according to the invention is a quaternary ammonium compound represented by the structure

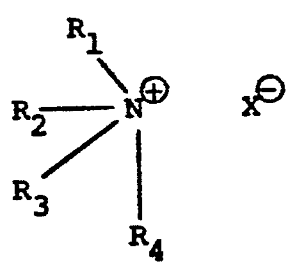

where
$R_1$, $R_2$, $R_3$ and $R_4$ can be
an alkyl radical of 1-12 carbon atoms;
a hydroxy ethyl radical;
a pyridyl radical;
or
a benzyl radical;
and
X can be Cl, Br, I, F or -OH.

Most quaternary compounds of this type are available in the marketplace. Those which are not can be made by the reaction of a tertiary amine and an alkyl halide as described in "Introduction to Organic Chemistry," Stretwieser and Heathcock, MacMillan Publishing Co., NY, 1976, page 777.

The quaternary compounds preferred for use according to the invention are tetramethyl ammonium bromide and tetraethyl ammonium iodide. Tetramethyl ammonium bromide is most preferred.

The reaction proceeds according to the equation

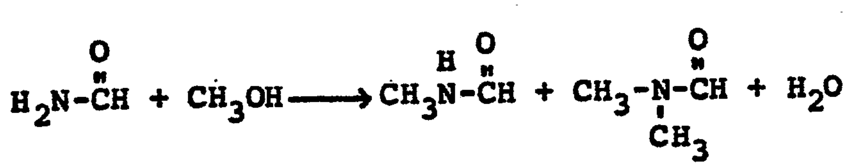

The reaction produces a mixture of MMF and DMF in proportions which can be varied as desired by changing the ratio of reactants (the reaction is stoichiometric, so use of two mols of methanol instead of one will produce more DMF instead of MMF).

The reaction can be run batchwise or continuously.

In the batch mode, the reactants are charged to a vessel in the desired proportion, together with the catalyst at a concentration of 0.1-5%, by weight of the reaction mass, preferably 0.5-1%. The reaction mass is then heated to and held at a temperature of 180-275°C, preferably 225°C. Lower temperatures than this can be used, but the reaction proceeds at a much slower rate, as would be expected. Temperatures above 275°C can also be used, but tend to promote the formation of undesirable by-products.

The reaction is run at autogenous pressure, normally 500-2000 psig (3447-13790 kPa).

When the reaction is complete, as determined by periodic sampling and analysis by gas chromatography, the vessel is opened and the contents removed. The products, MMF and DMF, and any unreacted reactants present, can then be separated by conventional fractional distillation, and the MMF and DMF products further refined in the customary way if this is necessary.

The process can be run continuously under the same conditions. Formamide and methanol are fed into a suitable reactor which contains the catalyst on a suitable support. The amount of catalyst to be used, and the residence time of the reactants in the reactor, are dictated by the size of the reactor and the conditions under which the reaction is to be run, and can be easily determined according to conventional chemical engineering principles. MMF and DMF are withdrawn from the top of the reactor and then conventionally separated and refined.

When this continuous mode is run in series with a continuous process for preparing formamide from ammonia and carbon monoxide, the combination provides an efficient two-stage process for making MMF and DMF directly from those reactants.

## EXAMPLES

Those skilled in the art will be able to practice this invention more easily after referring to the following illustrative examples.

These artisans will no doubt be able to compose numerous variations on the themes disclosed, such as changing the amounts of reactants slightly but insignificantly from those shown, substituting equivalent or nearly equivalent reactants for those shown, or adding innocuous substances. All these variations are considered to be part of the inventive concept.

In the Examples, all parts and percentages are by weight.

### Example 1

A reactor was charged with

| | |
|---|---|
| Methanol | 130 parts |
| Formamide | 30 parts |
| Tetramethyl ammonium bromide | 4 parts |

The temperature of the reaction mass was raised to 225°C over a period of 30 minutes and held at that temperature for 120 minutes, with stirring. Samples were withdrawn after 0, 20, 40, 60, 90 and 120 minutes and analyzed by gas chromatography for formamide, monomethylformamide and dimethylformamide content, as a percent of the reaction mass.

The results are shown in the following table:

| Minutes | % Formamide | % MMF | % DMF |
|---|---|---|---|
| 0 | 19.2 | 1.4 | 0.0 |
| 20 | 12.9 | 10.5 | 0.6 |
| 40 | 8.7 | 15.5 | 1.6 |
| 60 | 6.1 | 18.6 | 3.0 |
| 90 | 4.3 | 20.8 | 4.7 |
| 120 | 2.8 | 20.7 | 6.2 |

Example 2

The process of Example 1 was repeated, using tetraethyl ammonium iodide instead of tetramethyl ammonium bromide.

The results are shown in the following table:

| Minutes | % Formamide | % MMF | % DMF |
|---|---|---|---|
| 0 | 19.8 | 1.5 | 0.1 |
| 20 | 12.1 | 9.9 | 0.6 |
| 40 | 9.4 | 14.6 | 1.6 |
| 60 | 7.1 | 18.0 | 2.7 |
| 90 | 4.7 | 18.9 | 4.5 |
| 120 | 3.0 | 19.4 | 6.3 |

Example 3

The process of Example 1 was repeated, using as a charge

| | |
|---|---|
| Methanol | 140 parts |
| Formamide | 40 parts |
| Cetyl dimethyl benzyl ammonium chloride | 2 parts |

Samples were withdrawn from the reactor after 0, 60, 90, and 150 minutes and analyzed.
The results are shown in the following table:

| Minutes | % Formamide | % MMF | % DMF |
|---|---|---|---|
| 0 | 29.9 | 0.7 | 0.0 |
| 60 | 22.1 | 11.1 | 0.6 |
| 90 | 15.9 | 16.4 | 1.3 |
| 150 | 6.6 | 22.6 | 5.0 |

## Claims

1. A process for the preparation of monomethylformamide dimethylformamide by the catalytic reaction of formamide and methanol, characterised by using as the catalyst a quaternary ammonium compound represented by the structure

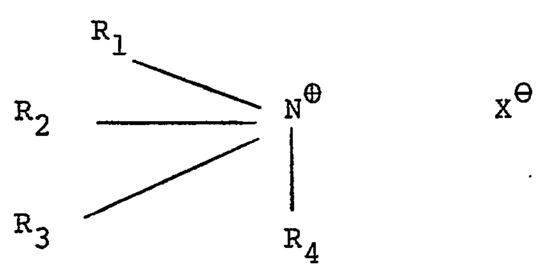

where
$R_1$, $R_2$, $R_3$ and $R_4$ can be
an alkyl radical of 1–12 carbon atoms;
a hydroxy ethyl radical;
a pyridyl radical;
or
a benzyl radical;

and
X can be Cl, Br, I, For –OH.

2. A process according to claim 1 in which the catalyst is tetramethyl ammonium bromide.

3. A process according to claim 1 in which the catalyst is tetraethyl ammonium iodide.

4. A process according to any one of the preceding claims in which the temperature is maintained in the range of 180 to 275°C and under autogenous pressure.

5. A process according to claim 4 in which the pressure is in the range of 500 to 2000 psig (3447 to 13790 kPa).

6. A process according to any one of the preceding claims in which the catalyst is used at a concentration of 0.5 to 1% by weigth of the reaction mass.

**Patentansprüche**

1. Verfahren zur Herstellung von Monomethylformamid und Dimethylformamid durch katalytische Reaktion von Formamid und Methanol, gekennzeichnet durch die Verwendung einer quaternären Ammoniumverbindung der Struktur

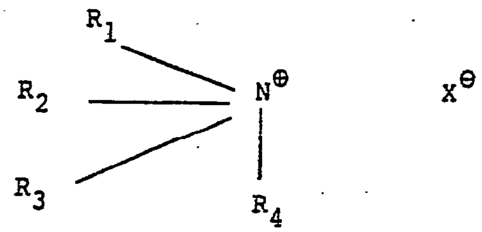

worin $R_1$, $R_2$, $R_3$ und $R_4$ ein Alkylrest mit 1 bis 12 Kohlenstoffatomen;
ein Hydroxyethylrest;
ein Pyridylrest; oder
ein Benzylrest sein können; und
X Cl, Br, I, F oder –OH sein kann, als Katalysator.

2. Verfahren nach Anspruch 1, worin der Katalysator Tetramethylammoniumbromid ist.

3. Verfahren nach Anspruch 1, worin der Katalysator Tetraethylammoniumiodid ist.

4. Verfahren nach einem der vorstehenden Ansprüche, worin die Temperatur unter autogenem Druck im Bereich von 180 bis 275°C gehalten wird.

5. Verfahren nach Anspruch 4, worin der Druck im Bereich von 500 bis 2000 psig (3447 bis 13790 kPa) liegt.

6. Verfahren nach einem der vorstehenden Ansprüche, worin der Katalysator in einer Konzentration von 0,5 bis 1 Gew.-% der Reaktionsmasse verwandt wird.

**Revendications**

1. Un procédé de préparation de monométhylformamide et de diméthylformamide par réaction catalytique de formamide et de méthanol, caractérisé en ce qu'on utilise, comme catalyseur, un composé d'ammonium quaternaire représenté par la structure

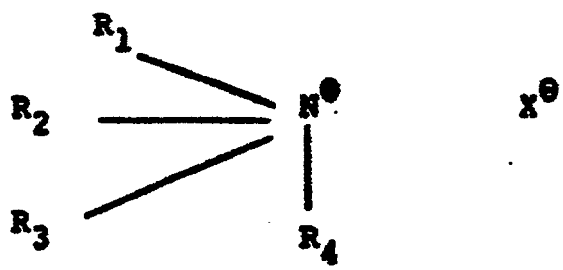

ou $R_1$, $R_2$, $R_3$, et $R_4$ peuvent être un radical alkyle de 1–12 atomes de carbone, un radical hydroxyéthyle, un radical pyridyle ou un radical benzyle, et X peut être Cl, Br, I, F ou –OH.

2. Un procédé selon la revendication 1, dans lequel le catalyseur est du bromure de tétraméthyl ammonium.

3. Un procédé selon la revendication 1, dans lequel le catalyseur est l'iodure de tétraéthyl ammonium.

4. Un procédé selon l'une quelconque des revendications précédentes, dans lequel on maintient la température dans l'intervalle de 180 à 275°C et sous la pression autogène.

5. Un procédé selon la revendication 4, dans lequel la pression est dans la gamme de 3447 à 13790 kPa (500 à 2000 psi relatives).

6. Un procédé selon l'une quelconque des revendications précédentes, dans lequel on utilise le catalyseur à une concentration de 0,5 à 1% en poids par rapport à la masse réactionnelle.